# EUROPEAN PATENT SPECIFICATION

(11) **EP 1 978 975 B1**
(45) Date of publication and mention of the grant of the patent: **24.12.2014**
(21) Application number: 07701070.0
(22) Date of filing: 01.02.2007
(51) Int. Cl.: A61K 31/765, A61P 41/00

(54) **COMPOSITION FOR INHIBITING ADHESION**
ZUSAMMENSETZUNG ZUR HEMMUNG VON ADHÄSION
COMPOSITION INHIBANT LES ADHÉRENCES

(30) Priority: 01.02.2006 KR 20060009849
(43) Date of publication of application: 15.10.2008
(73) Proprietor: Samyang Biopharmaceuticals Corporation, Seoul 110-470 (KR)
(72) Inventor: CHOI, In-Ja, Daejeon 305-503 (KR); SEO, Min-Hyo, Daejeon-city, 302-775 (KR); KIM, Bong-Oh, Daejeon-city, 300-810 (KR); SHIM, Myung-Seob, Seoul, 137-753 (KR)
(74) Representative: Novagraaf Technologies
(86) International application number: PCT/KR2007/000543
(87) International publication number: WO 2007/089103

(56) References cited:
- EP-A2- 0 369 764
- WO-A1-93/17669
- WO-A1-99/02168
- WO-A1-2006/014067
- KR-A- 20040 021 411
- KR-A- 20050 011 891
- US-A1- 2003 082 235
- US-A1- 2003 187 148
- US-A1- 2005 095 302
- US-B2- 6 923 986
- OH SE HEANG ET AL: "Prevention of postsurgical tissue adhesion by anti-inflammatory drug-loaded Pluronic mixtures with sol-gel transition behavior", JOURNAL OF BIOMEDICAL MATERIALS RESEARCH, vol. 72A, no. 3, 1 March 2005 (2005-03-01) , pages 306-316, XP002630802, ISSN: 0021-9304

## Description

### BACKGROUND OF THE INVENTION

### (a) Field of the Invention

This invention relates to a composition for inhibiting adhesion comprising a water-soluble and temperature-sensitive polymer showing a sol-gel phase transition dependant upon temperature,, wherein the polymer solution is subjected to phase-transition from a sol state to a gel state by body temperature, and is thus stably coated within tissues, thereby it functions as a barrier inhibiting adhesion, and as time lapses, it is degraded into a low molecular weight surfactant and a biodegradable single-molecular substance, whereby the degraded products exhibit secondary adhesion inhibiting effects.

More particularly, the invention relates to the use of a water-soluble and temperature-sensitive polymer showing a sol-gel phase transition dependant upon temperature in manufacturing a medicament for inhibiting adhesion of tissues, wherein the polymer is a temperature-sensitive multiblock copolymer where two or more identical or different block copolymers selected from polyethylene oxide (PEO)-polypropylene oxide (PPO)-polyethylene oxide (PEO) block copolymer and polyethylene oxide (PEO)-polybutylene oxide (PBO)-polyethylene oxide (PEO) block copolymer are linked by a biodegradable dicarboxyl group linker, and wherein said multiblock copolymer has an ionic group selected from the group consisting of a monovalent and divalent salt of -SO₃⁻; -PO₃²⁻, or -C(=O)-R-C(=O)-O⁻ at both terminal ends (where R is -(CH₂)ₘ- or an aryl of C_{m'}, m is an integer of 0 to 20, and m' is an integer of 6 to 12); wherein the polymer is subjected to phase-transition from a sol state to a gel state by the body temperature, stably coated within tissues, thereby functions as a barrier inhibiting adhesion, and as time lapses, degraded into a low molecular weight surfactant and a biodegradable single-molecular substance, whereby the degraded products exhibit secondary adhesion inhibiting effects.

### (b) Description of the Related Art

Adhesion refers to the phenomena that surrounding organs or tissues which are supposed to be separated from each other adhere together where fibrous tissues are excessively generated or bloods are run out and coagulated in the recovery course of wounds in inflammation, gash, friction, surgery cuts, etc. This adhesion causes serious problems, especially after surgery. The adhesion can be generally resulted from all kinds of surgery, and due to the adhesion, organs or tissues adjacent to surgery regions adhere to each other in the recovery process after surgery and accordingly, serious clinical sequelae may occur.

The kinds of sequelae that can be caused by the adhesion are very various. According to US statistics, it has been known that major symptoms generated by adhesion after surgery include intestinal obstruction at 49 % to 74%, infertility at 15 % to 20 %, chronic pelvic symptom at 20 % to 50 %, and enterobrosia in subsequent surgery at 19 % or so.

As methods for inhibiting adhesion, there can be mentioned methods of following the proceedings that should be cautioned during surgery such as, minimizing injury during surgery, washing away starch on the surface of gloves for surgery prior to their use, washing hands as often as possible, minimizing the frequency of use of devices for surgery, minimizing surgical operation so as to minimize foreign body reaction, etc., but such endeavors, in fact, do not completely prevent adhesion after surgery.

As methods of inhibiting adhesion based on the mechanism known as causing adhesion, there can be methods of activating tissue plasminogen activators in order to prevent the formation of fibrins and further, there are methods of preventing the formation of adhesion between adjacent tissues by forming physical barriers similar to surfactants during the recovery of tissue wounds, using a barrier known as another mechanism. In the case that such barriers are to be utilized, the barriers used have to be removed by degradation or absorption after the recovery of wounds over a certain period of time, and the materials for barrier themselves and degradation products thereof are required to be harmless to human. In addition, in view of inflammation reaction, together with endeavor of minimizing the extent of adhesion by using anti-inflammatory drugs and steroids to inhibit adhesion, there have been used methods of peeling the surfaces having adhesion formed thereon.

In order for barriers to be effective for adhesion inhibition, they are required to function as physical protective walls for tissues or organs during the recovery of wounds and at the same time, to prevent the formation of adhesion among adjacent tissues or organs without causing adverse effects on the recovery of the wounds. Also, after a certain period of time following treatments, they have to be eliminated by degradation or absorption in easy way and the materials for the adhesion barriers themselves and degradation products thereof should be harmless to human..

Adhesion barriers that are available as the protective walls can be classified into two types in their shapes: one is a solution-type including a gel-type and the other is a membrane-type including film, non-woven fabric, sponge, and so on.

Those known to be used as the materials of adhesion inhibitors are polyethylene glycol including poloxamer, polyethylene glycol, polysaccharides such as cellulose, chondroitin sulfate and hyaluronic acid, polylactic acid (PLA), collagen, fibrin and so on. So far, commercialized products based on the use of the above materials are oxidized regenerated cellulose, carboxyl methylcellulose, dextran (sulfate), hyaluronic acid and the like which are polysaccharides and polyethylene glycol, poloxamer and the like which are synthetic polymers. Of the materials for inhibiting adhesion, it has been known that celluloses and dextrans may generate foreign body reaction when inserted into a living body because they are not components constituting the living body although they are natural polymers. Furthermore, as there are no degradation enzymes targeted on those materials in the living body and their degradation in body is not thus possible, it has been known that additional treatments of converting them into absorbable forms in living body such as oxidation or hydrolysis are to be conducted.

US Patent No. 4,141,973 by Balazs, et al. discloses the use of hyaluronic acid as a main component for inhibiting adhesion. However, as the hyaluronic acid is readily degraded in a living body, it is relatively well dissolved and its half life in living body is relatively short, that is, 1 to 3 days so that it cannot be retained in body for the time necessary for inhibiting adhesion, it has a severe limit in functioning as an adhesion inhibitor.

Bromberg, et al. in US Patent No. 5,939,485 described that a polymer network has been developed which is responsive to environmental stimulus, such as pH, temperature and ionic strength. They used vinyl polymers, acryl polymers and urethanes that are non-degradable polymers in a living body as the structural components of the polymer network and used polyoxyalkylene polymers and cellulose polymers as the stimuli-sensitive polymers. However, if the non-degradable polymers as illustrated above are used as structural components, they may generate foreign body reaction because they are not degradable in living body and they have low biocompatibility.

US Patent No. 6,280,745 B1 by Flore, et al. describes a composition for the delivery of pharmaceutical agents for the purpose of preventing adhesion and a method for preventing adhesion after surgery using the same. The composition for the delivery of pharmaceutical agents comprises at least one constitutive polymer, modifier polymer and co-surfactant and can further comprise one selected from the group consisting of several pharmaceutical agents including antibiotic, anti-inflammatory agent. In this patent document, poly oxyalkylene block copolymers are illustrated as constitutive polymers, cellulose ethers, sodium carboxymethylcellulose and polyacrylates are illustrated as modifier polymers, and fatty acid soaps such as sodium oleate, sodium laurate, sodium caprate and sodium caprylate are illustrated as surfactants. Of the modifier polymers, as sodium carboxymethylcellulose is not a substance derived from a living body but prepared by processing the cellulose obtained from plants, it has been known to be able to generate foreign body reactions in a living body and likewise, other modifier polymers including the polyacrylates are not bio-derived substances and thus have low bio-compatibility, so they may generate foreign body reactions.

As another substance used as an adhesion inhibitor, poloxamer can be mentioned. Poloxamer, a polymer manufactured by BASF Company, has been known as a thermosensitive substance that exists in solution state at low temperature but is gelated as temperature increases (see US Patent No. 4,188,373, US Patent No. 4,478,822 and US Patent No. 4,474,751). US Patent No. 5,939,485 by Bromberg, et al. describes that these poloxamers are substances capable of reversible gelation by stimulus of pH, temperature, ionic strength. Further, Steinleitner, et al. published an evaluation of the anti-adhesion efficacy of fluid gels having poloxamers as a basic composition [Fertility and sterility 57(2): 305 (1992)].

Generally known poloxamers have the structure of polyethylene oxide (PEO)-polypropylene oxide (PPO)-polyethylene oxide (PEO). For example, Poloxamer 407 has a gelation temperature of 25°C or so and its gelation is influenced by factors such as poloxamer grade, concentration, pH and additive. In addition, the melting temperature of Poloxamer 407 is 56 °C and its specific weight is 1.05. However, as this poloxamer forms polymer gel in aqueous solutions but it is easily dissolves in water, it has the drawback that it does not retain its gel state at a certain area for a time sufficient to inhibit adhesion.

US Patent No. 6,316,011 B1 by Ron, et al. describes a heat-reversible composition with a polymer or oligomer for modification at its end portion. The heat-reversible composition comprises PPG-PEG-PPG block copolymers and biocompatible polyvinylcarboxylic acid was used as the polymer for modification located at the end. Several pharmaceutical drugs that can be used to inhibit adhesion can be added and mixed into the composition. In this patent document, the bioadhesion polyvinylcarboxylic acid comprises acrylic acid and methacrylic acid, which have low biodegradability and biocompatibility and thus may induce foreign body reaction in living body.

Up to now, the studies about adhesion inhibitors using various materials have been conducted seriously and some of them succeeded to the marketing, but the current products are Interceed by Ethicon Company and Separafilm by Genzyme Company at the most. However, it has been reported that such adhesion inhibitors in the form of film were not well attached to the surface of a living body when applied to interior organs, and even though they succeeded in attachment, they were not located in their original place due to the movement of organs and they were recognized as foreign materials by the tissues themselves and thus conglomerated with each other and consequently, they showed unsatisfactory adhesion inhibiting effects. Nevertheless, no alternate materials capable of replacing them have been developed so far. In fact, these products in the form of film are used only in limited areas such as obstetrics and gynecological surgery or spine surgery.

In order to overcome such problems, Flowgel (Mediventures) comprising carboxymethylcellulose, dextran 70 and Poloxamer 407 consisting of PEO-PPO-PEO in the form of gel, Adcon-L (Gliatech) based on polylactic acid, Intergel (Lifecore Biomedical) based on hyaluronic acid, AdbA (Amitie) using natural polymers and Spraygel (Confluent Surgical) based on polyethylene oxide in the form of spray have been developed and some of them were placed on the market. The period required for the recovery of wounds varies, depending on the degree of the wounds, but it generally takes 7 days or so. However, in the case of the adhesion inhibitors in the form of gel developed above, as they are melted and discharged before the wounds are recovered, that is, they do not retain their shapes within the wounded tissues for a sufficient time, they fail to fully exert adhesion inhibiting effects and also their ingredients cause foreign body reaction, making it difficult to place them on the market.

US 2003/187148 relates to water-soluble and temperature sensitive multiblock copolymers showing a sol-gel phase transition at a specific temperature for inhibiting the adhesion of tissues. The block copolymers comprise polyethylene oxide (PEO)-polypropylene oxide (PPO)-polyethylene oxide (PEO) repeating units and are linked by a dicarboxyl group chain extender. Despite showing exceptional reverse thermal gelation properties, low viscosity at room temperature and high viscosity above room temperature (32-40°C), the multiblock copolymer is not suitably adapted to effectively control the release of bioactive materials and show an improved drug release profile, as is required in order to better solve the problem of tissue adhesion.

US 2005/095302, relates to the same multiblock copolymer and is centered on the methods of treatment using this polymer to reduce tissue adhesions in subjects in need. Since the polymer is the same, this document does not address the need for an improved drug release profile from the gelled polymer *in vivo,* either.

If adhesion inhibitors in the form of gel which complement the drawbacks of the adhesion inhibitor in the form of film and have more ideal conditions are developed, the application fields of the adhesion inhibitors that are currently used in a very restrictive way in surgical operation fields can be extended to where the adhesion inhibitors in the form of film could not be applied and it is expected that it can be used in surgical fields where the application of the adhesion inhibitors was not possible to date. Further, in view of transition trend in surgery methods in the direction for reducing infection at its maximum, it is expected that the adhesion inhibitors in the form of gel can make surgery easy and reduce the risk associated with infection at the least while they still have excellent adhesion inhibiting effects, compared with regional treatment based on adhesion inhibitors in the form of film.

Therefore, it can be said that up to now, no adhesion inhibitors having ideal conditions and successful effects have been developed and the development of such ideal and effective adhesion inhibitors is desperately needed.

### SUMMARY OF THE INVENTION

In order to solve the aforementioned problems and comply with the requirements, it is an object of the present invention to provide an adhesion inhibitor in the form of a gel comprising a temperature-sensitive polymer with biocompatibility that it can be degraded in the body, absorbed and excreted and capable of gelation by body temperature and more particularly, an ideal adhesion inhibitor in the form of gel which can be safely retained within a desirable location in body for a certain period of time, the degradation products of which can be easily absorbed and excreted when it is degraded in the body wherein the substances generated from the degradation process function as a surfactant, conferring synergy effects on adhesion inhibition, and that can exert adhesion inhibiting effects for a long time.

### BRIEF DESCRIPTION OF THE DRAWINGS

Fig. 1 (a) is a graph showing a ¹H-NMR spectrum of Poloxamer 407 and Fig. 1 (b) is a graph showing a ¹H-NMR spectrum of the multiblock copolymers of Preparation Example 1 which have been linked via a succinyl group.
Fig. 2 is a ¹H-NMR spectrum of Poloxamer 407 after treatment with TMS-Cl/pyridine.
Fig. 3 is a ¹H-NMR spectrum of Poloxamer 407 after substitution of the ends of Poloxamer 407 with succinic acid and then treatment with TMS-Cl/pyridine.
Fig. 4 is a ¹H-NMR spectrum after the treatment of the multiblock copolymers of Preparation Example 1 with TMS-Cl/pyridine.
Fig. 5 is a graph showing the results of viscosity determination of Poloxamer 407 by Brookfield viscometer.
Fig. 6 is a graph showing the results of viscosity determination of MBP-36 prepared in Preparation Example 1 by Brookfield viscometer.
Fig. 7 is a graph showing the results of viscosity determination of MBP-29 prepared in Preparation Example 5 by Brookfield viscometer.
Fig. 8 is a graph showing the results of viscosity determination of MBP-22 prepared in Preparation Example 6 by Brookfield viscometer.
Fig. 9 is a graph showing the results of viscosity determination of MBP-42 prepared in Preparation Example 7 by Brookfield viscometer.
Fig. 10 is a graph showing the results of viscosity determination of MBP-77 according to ethanol concentrations in a mixed solvent by Brookfield viscometer.
Fig. 11 is a photograph showing adhesion on 7 days after surgery in the control group where no treatment was given after the surgery.
Fig. 12 is a photograph showing adhesion on 7 days after surgery in the case treated with MBP-36 prepared in Preparation Example 1 of the present invention in the amount of 3 % by weight.
Fig. 13 is a photograph showing adhesion on 7 days after surgery in the case treated with MBP-36 prepared in Preparation Example 1 of the present invention in the amount of 5 % by weight.
Fig. 14 is a photograph showing adhesion on 7 days after surgery in the case treated with MBP-36 prepared in Preparation Example 1 of the present invention in the amount of 7 % by weight.
Fig. 15 is a photograph showing adhesion in the case treated with the solution of MBP-36 prepared in Preparation Example 1 of the present invention to which carboxylmethylcellulose was added.
Fig. 16 is a photograph showing adhesion in the case treated with MBP-53 prepared in Preparation Example 4 of the present invention in the amount of 1 % by weight.
Fig. 17 is a photograph showing adhesion in the case treated with MBP-53 prepared in Preparation Example 4 of the present invention in the amount of 3 % by weight.
Fig. 18 is a photograph showing adhesion in the case treated with MBP-53 prepared in Preparation Example 4 of the present invention in the amount of 5 % by weight.
Fig. 19 is a photograph showing adhesion in the case treated with MBP-53 prepared in Preparation Example 4 of the present invention in the amount of 7 % by weight.
Fig. 20 is a photograph showing adhesion in the case treated with MBP-53 prepared in Preparation Example 4 of the present invention in the amount of 10 % by weight.
Fig. 21 is a photograph showing adhesion in the case treated with Poloxamer F-407 solution.
Fig. 22 is a graph showing changes in body weight on 7 days after surgery in the animals treated with the adhesion inhibitor containing MBP-36 prepared in Preparation Example 1 of the present invention.
Fig. 23 is a graph showing changes in body weight on 7 days after surgery in the animals treated with the adhesion inhibitor containing MBP-53 prepared in Preparation Example 4 of the present invention.

### DETAILED DESCRIPTION OF THE PREFERRED EMBODIMENTS

A more complete appreciation of the invention, and many of the attendant advantages thereof, will be readily apparent as the same becomes better understood by reference to the following detailed description.

This invention relates to the use of a water-soluble and temperature-sensitive polymer showing a sol-gel phase transition dependent on temperature as defined in claim 1 in the manufacture of a medicament for inhibiting the adhesion of tissues,
wherein the polymer is subjected to phase-transition from a sol state to a gel state by body temperature, is thus stably coated within tissues, thereby it functions as a barrier inhibiting adhesion, and as time lapses, it is degraded into a low molecular weight surfactant and a biodegradable single-molecular substance, whereby the degraded products exhibit secondary adhesion inhibiting effects.

The surfactants may be a unit polymer of low molecular weight constituting the polymers of the invention and the single molecule substances may be a linker linking the unit polymers of low molecular weight. The content of the polymers is preferably 0.1 to 40 % by weight of the total composition and the composition for inhibiting adhesion is preferably present in a gel type.

An ideal adhesion inhibitor has the following conditions:
1) It should have adhesion inhibiting effects on tissues or organs near wounds.
2) It should be water-soluble.
3) It should be harmless to human and easily degraded in a human body so as to be readily absorbed and excreted.
4) It should not generate any foreign body reaction.
5) It should be retained within tissues or organs for a certain period of time necessary for the recovery of wounds.
6) The application and treatment thereof should be easy.

In order to satisfy the conditions as mentioned above, a gel type is most suitable.

The described polymers, the adhesion inhibitors in the form of gel type sensitive to temperature, are gelated by body temperature and safely retained within organs for a certain period of time, thereby not only functioning to inhibit adhesion but also they can be absorbed into body or degraded, and after degradation in living body, the degradation products are also easily absorbed into the body and excreted and at the same time, the substances generated from the degradation process function to inhibit adhesion as a surfactant, thereby maintaining adhesion inhibiting effects for a long time. Furthermore, they can minimize a foreign body sensation in a living body and can be applied by being locally sprayed or coated onto wounded regions during surgery and/or after surgery and accordingly, they can be handled in an easy and convenient way.

The polymers of the invention have the above characteristics and are temperature-sensitive multiblock copolymers wherein two or more identical or different block copolymers selected from polyethylene oxide (PEO)-polypropylene oxide (PPO)-polyethylene oxide (PEO) block copolymer and polyethylene oxide (PEO)-polybutylene oxide (PBO)-polyethylene oxide (PEO) block copolymer are linked by a biodegradable dicarboxyl group linker, and wherein said multiblock copolymer has an ionic group selected from the group consisting of a monovalent and divalent salt of -SO₃⁻, -PO₃²⁻, or - C(=O)-R-C(=O)-O⁻ at both terminal ends, where R is -(CH₂)ₘ- or an aryl of C_{m'}, m is an integer of 0 to 20, and m' is an integer of 6 to 12.

The multiblock copolymers contained in the composition for inhibiting adhesion of the invention are those fully satisfying the conditions as adhesion inhibitors, and detailed description for them is as follows.

According to the context of the present, a "block copolymer" refers to a basic unit where polyethylene oxide (PEO) block that is a hydrophilic block is linked to polypropylene oxide (PPO) or polybutylene oxide (PBO) block that is a hydrophobic block, which is then linked to polyethylene oxide (PEO) block and it is represented by a PEO-PPO-PEO block copolymer or PEO-PBO-PEO block copolymer.

According to the context a "multiblock copolymer" refers to a polymer having a block copolymer where polyethylene oxide (PEO) block that is a hydrophilic block is linked to polypropylene oxide (PPO) or polybutylene oxide (PBO) block that is a hydrophobic block which is then linked to polyethylene oxide (PEO) block, as a basic unit, the two or more block copolymers being linked by a dicarboxyl group linker.

For example, the present discloses a composition for inhibiting adhesion comprising a multiblock copolymer where two or more identical or different block copolymers selected from the group consisting of a PEO-PPO-PEO block copolymer and PEO-PBO-PEO block copolymer are linked by a dicarboxyl group linker, wherein the multiblock copolymer in the above is a temperature sensitive polymer and exists in its gel state within body temperature ranges because its phase transition temperature is lower than the body temperature, its retainment period in polymer aqueous solution environment of low concentration can be improved over a few days because its weight average molecular weight is relatively high, it is water-soluble, and it is easily degraded in a living body so as to be absorbed and excreted because it is linked by biodegradable linkers. The present invention provides a new use of the multiblock copolymer comprising PEO-PPO (or PBO)-PEO block copolymers sensitive to temperature as an effective adhesion inhibitor.

The multiblock copolymer used in the invention, in the composition for inhibiting adhesion, has an average molecular weight within the ranges of 1,000 to 20,000 daltons, it is in the form where two or more identical or different block copolymers selected from the group consisting of PEO-PPO-PEO and PEO-PBO-PEO block copolymers having identical or different molecular weights are multiblock-copolymerized by being linked by a dicarboxyl group linker within the above weight ranges, and its total weight average molecular weight is 25,000 to 1,000,000 daltons, preferably 50,000 to 500,000 daltons. The multiblock copolymers of the invention can exhibit a sol-gel phase transition at a low concentration by having increased molecular weight by being multiblocked with a dicarboxyl group linker and accordingly, they have the merit that they can show continuance for a sufficient period of time.

The molecular weight ratio of PEO and PPO or PBO of the multiblock copolymers can vary within the limits at which the polymers maintain their water-soluble property, can be about 0.2:1 to about 40:1, preferably 1:1 to 7.5:1, and more preferably 1:1 to 5:1, and the PEO block can be included in the amount of 10 to 85 % by weight, preferably 40 to 85 % by weight of the PEO-PPO or PBO-PEO units

The polyethylene oxide (PEO) block in the block copolymers can comprise about 2 to 2000, preferably about 5 to 500, and more preferably about 80 to 120 ethylene oxides, and the number of the each ethylene oxides constituting the two PEO blocks contained in the respective block copolymers can be either the same or different.

The polypropylene oxide block or polybutylene oxide block can comprise 2 to 2000, preferably about 20 to 500, and more preferably about 30 to 250 propylene oxides or butylene oxides.

The multiblock copolymers of the invention are formed by linking two or more randomly selected from the group consisting of PEO-PPO-PEO block copolymers and PEO-PBO-PEO block copolymers, and the two or more block copolymers can be either the same or different from each other.

The dicarboxyl group linker used for the linkage is a pharmacologically acceptable one, which can be one or more dicarboxylic acids selected from the group consisting of alkyldicarboxylic acids such as oxalic acid, succinic acid, glutaric acid, adipic acid, sebacoyl acid, malonic acid, suberic acid and dodecanonic acid; unsaturated dicarboxylic acids such as fumaric acid and maleic acid; and allyldicarboxylic acids such as phthalic acid and terephthalic acid and of them, oxalic acid, succinic acid, glutaric acid, adipic acid, sebacoyl acid, fumaric acid or maleic acid is preferable.

The dicarboxyl group linkers are linked to hydroxyl groups at the both ends of the block copolymers via an ester bond, which allows the polymers to be easily degraded into carboxylic acids and PEO-PPO (or PBO)-PEO block copolymers by hydrolysis or the action of enzymes in aqueous solutions or a living body, giving the multiblock copolymers a water-soluble property. The degraded PEO-PPO (or PBO)-PEO block copolymers function as a surfactant and can thus exert secondary adhesion inhibiting effects. Thus, as the multiblock copolymers are degraded into block copolymers with surface activity having a small molecular weight and dicarboxylic acids by hydrolysis in a body, the invention can provide continuous adhesion inhibiting effects by the continuous release of the block copolymers even after the polymers having a high molecular weight are degraded.

The multiblock copolymers disclosed may have a structure of the following formula 1:

M-X-O-[PEO-Y-PEO-C(-O)-R-C(=O)-O]ₙ-PEO-Y'-PEO-O-X-M

wherein, PEO is a polyethylene oxide,
Y and Y' are each independently polypropylene oxide (PPO), polybutylene oxide (PBO), or a combination of PPO and PBO,
X is an anion group,
n is an integer of 1 to 100,
R is -(CH₂)ₘ-, or an aryl of C_{m'},
m is an integer of 0 to 20, and m' is an integer of 6 to 12,
M is a cation group, and preferably, M is selected from the group consisting of Li, Na, K, Ag, Au, Ca, Mg, Zn, Fe, Cu, Co, and Ni, and
.

Preferably, the multiblock copolymers of the invention can be represented by formula 2:

M-X-O-[PEO-Y-PEO-C(=O)-R-C(=O)-O]ₐ-PEO-Y'-PEO-O-X-M

wherein, PEO is a polyethylene oxide,
Y and Y' are each independently polypropylene oxide (PPO), polybutylene oxide (PBO), or a combination of PPO and PBO,
X is, -SO₃-, -PO₃²⁻, or -C(=O)-R-C(=O)-O-
n is an integer of 1 to 100,
R is -(CH₂)ₘ-, or an aryl of C_{m'},
m is an integer of 0 to 20, and m' is an integer of 6 to 12, and
M is a monovalent or divalent cation group and preferably, M is selected from the group consisting of Li, Na, K, Ag, Au, Ca, Mg, Zn, Fe, Cu, Co, and Ni, and.

The both ends of the multiblock copolymers of the invention are salts comprising anion groups selected from -SO₃⁻,-PO₃²⁻, or -C(=O)-R-C(=O)-O⁻ and the cation groups forming salts in response to the anion groups can be monovalent cation groups such as Li, Na, K, Ag, and Au and divalent cation groups such as Ca, Mg, Zn, Fe, Cu, Co, and Ni.

In an embodiment of the present invention, the basic unit of the multiblock copolymers, PEO-PPO (or PBO)-PEO, can be commercially available poloxamers.

The poloxamer is a compound where hydrophilic block, polyethylene oxide (PEO) and hydrophobic block, polypropylene oxide (PPO) are linked via ether bond in the triple block form of PEO-PPO-PEO, has a weight average molecular weight of 1,000 to 20,000 daltons and is a block copolymer with hydroxyl groups at its terminals. Specifically, there can be used Poloxamer 188 (Pluronic® F-68), Poloxamer 407 (Pluronic® F-127) and so on.

A composition for inhibiting adhesion comprising a multiblock copolymer where Poloxamer 407 is linked via ester bond by a dicarboxylic acid in an amount of 1 to 20 % by weight in the state of an aqueous solution, wherein the weight average molecular weight of the multiblock copolymer is 50,000 to 500,000 daltons, and it is gelated at 15 °C and higher in an aqueous solution is also hereby disclosed.

The poloxamers can be used after purification or without purification to prepare the polymers of the invention and if purification is performed, it is easy to prepare polymers having high molecular weight. The purification of poloxamers can be performed by dissolving it in methylenechloride and then precipitating it in hexane or by layer separation methods in n-propanol/H₂O solvent as disclosed in US Patent No. 5,800,711.

The multiblock copolymers can be prepared by the following methods. First, diluted dicarboxylic acid dichloride is added to the block copolymers of PEO-PPO (or PBO)-PEO in a suitable amount depending on the type of the terminal groups present at the both ends of the polymers and then coupling reaction is carried out for a certain period of time, thereby to obtain a product with increased molecular weight. The obtained product is purified by adding it to ether to precipitate polymers, which are then dissolved in methanol, followed by the addition of ether to precipitate polymers. The both ends of the multiblock copolymers can be introduced to the precipitated polymers by all the known methods.

Synthesis of the multiblock block copolymers from the poloxamers can be identified by nuclear magnetic resonance (NMR). A peak that does not exist in the NMR spectrum of the monomer poloxamer alone used in the reaction, which is the peak of terminal -CH₂CH₂- generated by the bond of carboxylic acid and hydroxyls at the both ends of the poloxamer, is generated near 4.2 ppm in the multiblock copolymers synthesized using dicarboxylic acid as a linker. Further, the introduction of dicarboxylic acid can be verified by reacting the synthesized poloxamer oligomers with trimethylsilylchloride (TMS-Cl) in the presence of triethylamine and then determining their NMR spectrum. If the terminal group is a hydroxyl, the signal of trimethylsilyl proton is observed at 0.12 ppm and if it is a carboxyl terminal group, this peak is observed at 0.3 ppm. Based on them, the synthesis of poloxamer oligomers can be identified.

The content of the multiblock copolymers in the composition for inhibiting adhesion can be 0.1 to 40 % by weight, preferably 1 to 30 % by weight and most preferably 1 to 20 % by weight. If the content of the multiblock copolymers is less than the above ranges, it is difficult to obtain effective adhesion inhibiting effects and to maintain gel state within body temperature ranges, and if it is in excess of the above ranges, handling is not easy and effect increase versus contents is slight, cost-ineffective in the light of the fact that it involves a large amount of polymers. The multiblock copolymers used in the invention has a sol-gel phase transition temperature within the ranges of 10 to 35 °C in the state of aqueous solution of 0.1 to 40 % by weight concentrations and accordingly, they can maintain their gel state within body temperature ranges. In this regard, as they can be retained in attachment to the regions as desired for 7 days or longer when applied to wounded regions, they have the merits that they remain in living body for a sufficient period of time. In general, when considering that the period during which adhesion between organs and/or tissues occurs is within 7 days after surgery, the fact that the multiblock copolymers can be retained at the desired position in body for 7 days or longer is very important to inhibit adhesion.

The existing poloxamer (PEO-PPO-PEO) becomes the sol state at temperatures lower than 20°C when the concentration of the poloxamers in aqueous solutions is 20 to 40 % by weight and it becomes the gel state at 20°C or higher and can exist in the gel state within the body temperature ranges. On the contrary, as the multiblock copolymers become the sol state at temperatures lower than 20°C even in the state of aqueous solution having relatively low concentrations of 1 to 20 % by weight and maintain the gel state at 20°C or higher, it can be maintained in the gel state within the body temperature ranges with low concentration. Such characteristics are in accord with the requirements of adhesion inhibitors that should be retained for 3 days or longer with a one-time dose.

The solvent contained in the composition for inhibiting adhesion is a solvent capable of dissolving the multiblock copolymers and there can be used those selected from the group consisting of a distilled water, water for injection, physiological saline, 0.1 to 50 v/v% alcohol aqueous solution, natural intraperitoneal solution and artificial intraperitoneal solution having the compositions of the natural intraperitoneal solution. The alcohol is ethanol, 1,2-propyleneglycol, glycerol, polyethyleneglycol 300 or polyethyleneglycol 400.

Preferably, the invention employs 0.1 to 50 v/v% alcohol aqueous solution as a solvent. Although aqueous solutions that do not contain ethanol show excellent adhesion inhibiting effects, there are matters to be considered in terms of the concentration of the polymers and phase transition change pattern. If the concentration of the polymers is high, adhesion inhibiting effects are improved, but they exhibit phase transition within narrow ranges due to their high viscosity and thus they easily become the gel when handled as adhesion inhibition solutions so that their handling might be difficult. On the contrary, in the case of solutions having low concentrations, they can be handled easily due to their broad phase transition ranges but their adhesion inhibiting efficiency can be low.

Meanwhile, in the case of the aqueous solutions that do not contain ethanol, as temperature increases, the viscosity of the solutions exhibit sudden phase transition at a certain temperature whereas in the case of using alcohol aqueous solutions as a solvent, as the content of ethanol increases, the viscosity of the solutions exhibits phase transition over some broad temperature ranges. That is, the aqueous solutions that do not contain ethanol involve troubles to be stored in an ice container when stored or treated at room temperature for a long time because the viscosity of the solutions increases and they might be difficult to be handled. In particular, when handled at temperatures near phase transition, the solutions are difficult to be handled due to sudden phase transition phenomena. Accordingly, as the mixed solvents with ethanol provide phase transition over broad temperature ranges, it is very easy to handle polymer solutions. Also, the use of ethanol containing aqueous solutions can complement the drawbacks of solutions of which the viscosity is too high to be handled. Thus, the polymer solutions having the same amounts can be readily handled by including ethanol, thereby lowering the viscosity of the solutions. Furthermore, when coated into body, the solutions can be easily gelated due to the absorption and evaporation of ethanol.

The multiblock copolymers used for manufacturing a medicament for inhibiting adhesion of tissue according to the invention are characterized in that they can comprise only the single substances of PEO-PPO-PEO or PEO-PBO-PEO block copolymers, they do not necessarily need to comprise active ingredient other than the block copolymers, they are degraded into PEO-PPO-PEO or PEO-PBO-PEO block copolymers of the single substances and the linker, dicarboxylic acid, and the degradation products function as another adhesion inhibiting mechanism.

As described above, since the composition for inhibiting adhesion of the invention comprises multiblock copolymers that exhibit sufficient temperature sensitivity merely with these single substances, it is characterized in that the mere use of the multiblock copolymers without other natural polymer components exhibiting foreign body sensation enables a sol-gel phase transition within the body temperature ranges. The composition for inhibiting adhesion of the invention is characterized in that it exhibits adhesion inhibiting performance of tissues and/or organs merely with the use of the multiblock copolymers without the addition of antiphlogistic agent and other additives and even after they are degraded in body, the degradation products function as a surfactant having a secondary adhesion inhibiting effects.

The composition for inhibiting adhesion comprising the multiblock copolymers may further comprise pharmaceutical drugs having independent pharmacological effects. Such pharmaceutical drugs can be those that adjust the gelation properties of the composition to be coated onto wounded regions or function as an active component for treating the wounds. Available pharmaceutical drugs are anti-thrombogenesis agents such as heparin or tissue plasminogen activator, non-steroid anti-inflammatory drugs such as aspirin, ibuprofen and ketoprofen, hormone chemostatic factors, analgesics or anesthetics.

The composition for inhibiting adhesion is applicable to laparotomy and laparoscopic surgery and also can be applied to general surgical fields including not only existing peritoneal surgery, bladder or gynecological surgery and spine surgery but also heart surgery, rectal surgery, dental surgery and various kinds of plastic surgery.

The composition is applicable in various forms such as a tube, cream, syringe and spray, depending on its use, and it can inhibit the adhesion of tissues by injection, coating or spray onto wounded regions and then gelation.

The aqueous solution composition is a biodegradable polymer substance and thus cannot be stored in the state of its aqueous solution. Therefore, a container comprising the multiblock copolymers and a container comprising the aqueous solution are packaged separately and then when in use, they are mixed and dispersed to become a polymer aqueous solution. The multiblock copolymers filled in the container can be those prepared by lyophilization.

The content of the composition for inhibiting adhesion to be applied can vary by polymer composition, molecular weight, concentration, period required for adhesion inhibition, region to be applied in body, patient's condition and so on.

The invention will be further described by particular embodiments, which are provided to illustrate the invention.

### EXAMPLES

### Preparation Example 1. Synthesis of Multiblock Copolymer Composed of Poloxamer 407 Using Succinyl Dichloride

10 g of Poloxamer 407 (Pluronic ® F-127, BASF, molecular weight: 12,500 daltons, PEO:PPO=101:56) was poured into a 100-ml one-neck round bottom flask and heated in a boiling oil heated at 120°C and then, moisture contained in the polymers was eliminated for 2 hours while pressure was being reduced. After the removal of pressure reduction, the reaction temperature was set to 100°C with nitrogen being flowed and then 100 ml of acetonitrile was added to the flask. The reaction flask was equipped with a dean stark and cooling unit.

After the moisture within the reactants was completely removed by eliminating 20 ml of acetonitrile that was distilled out through the dean stark, 1 equivalent of succinyl dichloride was added to the reservoir of the dean stark device and reacted for 24 hours. After 24 hours, 96 ul of succinyl chloride was added again to the dean stark device so as to substitute the terminal group of the synthesized poloxamer oligomers with carboxyl group and the reaction was carried out for 24 hours. The synthesized poloxamer oligomers were precipitated in 1 L of diethylether and filtered, thereby obtaining a product. The obtained product was dissolved again in 16 ml of methanol and then precipitated in diethylether and filtered. This purification process was carried out twice. The purified product was dried under vacuum, affording poloxamer oligomers with narrow molecular weight distribution.

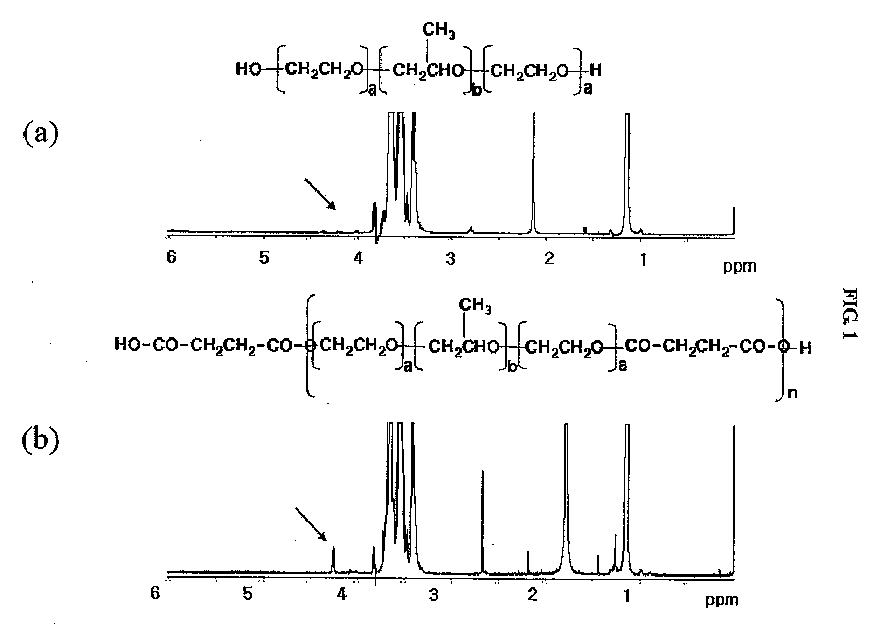

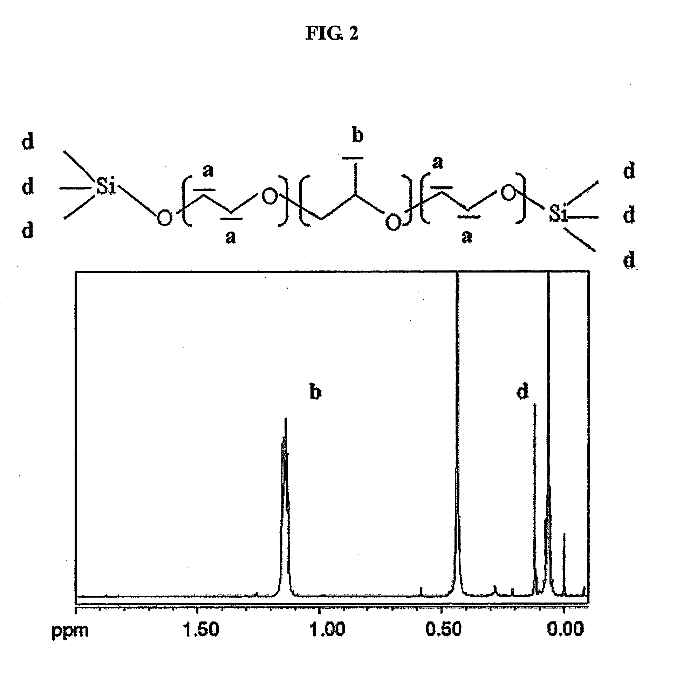

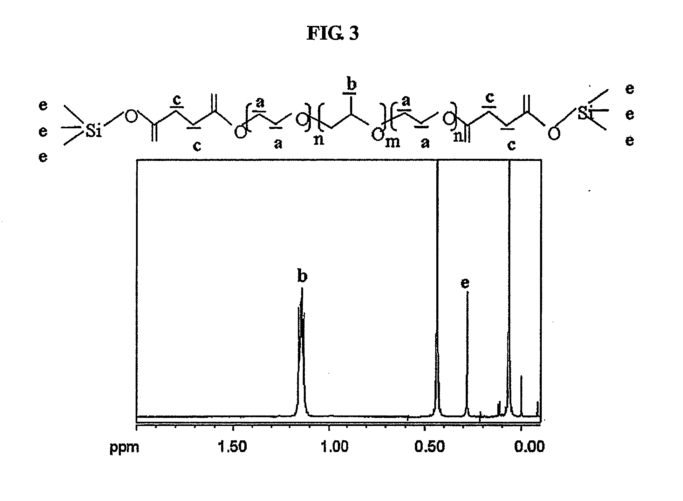

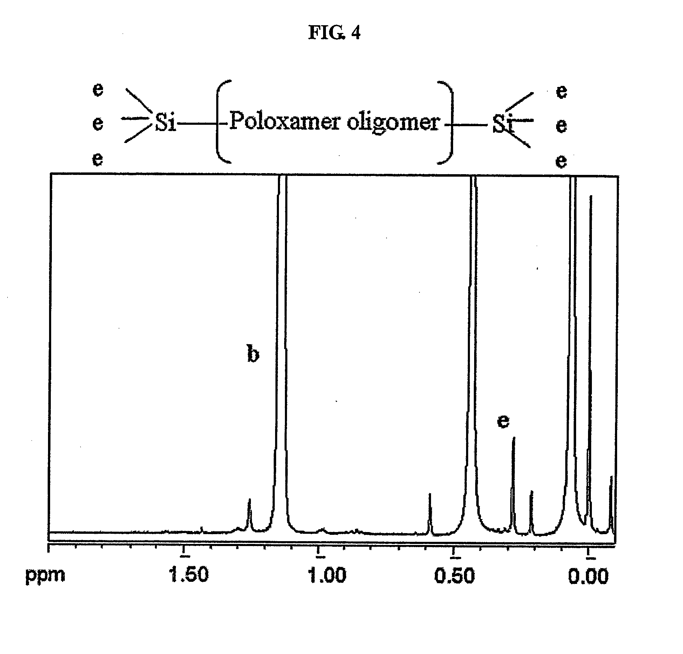

To identify whether or not the produced substances are the multiblock copolymers of poloxamer, nuclear magnetic resonance (NMR) was used. As a result, a peak that did not exist in the NMR spectrum of the monomer poloxamer alone used in the reaction, which is the peak of terminal -CH₂CH₂- generated by the bond of carboxylic acid and the hydroxyls at the both ends of the poloxamer, was generated near 4.2 ppm in the multiblock copolymers synthesized using dicarboxylic acid as a linker (Fig. 1). Further, the introduction of dicarboxylic acid was identified by reacting the synthesized poloxamer oligomers with trimethylsilylchloride (TMS-Cl) in the presence of triethylamine and then determining their NMR spectrum. If the terminal group is a hydroxyl, the signal of trimethylsilyl proton is observed at 0.12 ppm and if it is a carboxyl terminal group, this peak is observed at 0.3 ppm. Thus, the synthesis of the synthesized poloxamer oligomers could be identified (Fig. 2 to Fig. 4).

The thus obtained product was named MBP-36, of which the weight average molecular weight was 230,000 daltons. All the average molecular weights throughout the examples of the present invention including this example were determined by Gel Permeation Chromatography (GPC).

### Preparation Example 2. Synthesis of Multiblock Copolymers Composed of Poloxamer 188 Using Adipoyl Dichloride

With the exception that Poloxamer 188 (Pluronic® F-68, BASF, molecular weight: 8,000 daltons, PEO:PPO=80:27) was used as a basic unit of multiblock copolymer and adipoyl dichloride was used as a dicarboxylic acid linker, multiblock copolymers having an average molecular weight of 160,000 were synthesized in accordance with the same methods as used in Preparation Example 1. Through the peak generation near 4.2 ppm and terminal peak analysis due to the introduction of dicarboxylic acid using nuclear magnetic resonance, the produced multiblock copolymers were identified as the poloxamers linked by dicarboxylic acid.

### Preparation Example 3. Synthesis of Multiblock Copolymer Composed of Poloxamer 237 Using Succinyl Dichloride

With the exception that Poloxamer 237 (BASF, molecular weight: 7,000 daltons, PEO:PPO=64:37) was used as a basic unit of multiblock copolymer, multiblock copolymers having an average molecular weight of 150,000 were synthesized in accordance with the same methods as used in Preparation Example 1 using succinyl dichloride as a dicarboxylic acid linker. Through the peak generation near 4.2 ppm and terminal peak analysis due to the introduction of dicarboxylic acid using nuclear magnetic resonance, the produced multiblock copolymers were identified as the poloxamers linked by dicarboxylic acid.

### Preparation Example 4. Synthesis of Multiblock Copolymers Composed of Poloxamer 407 Using Succinyl Dichloride

The same methods as used in Preparation Example 1 were carried out using Poloxamer 407 (molecular weight: 12,500 daltons, PEO:PPO=101:56) as a basic unit of multiblock copolymer and using succinyl dichloride as a dicarboxylic acid linker, thereby synthesizing multiblock copolymers having an average molecular weight of 130,000, which was named MBP-53. Through the peak generation near 4.2 ppm and terminal peak analysis due to the introduction of dicarboxylic acid using nuclear magnetic resonance, the produced multiblock copolymers were identified as the poloxamers linked by dicarboxylic acid.

### Preparation Example 5. Synthesis of Multiblock Copolymers Composed of Poloxamer 407 Using Oxalic Chloride

With the exception that Poloxamer 407 (molecular weight: 12,500 daltons, PEO:PPO=101:56) was used as a basic unit of multiblock copolymer and oxalic chloride was used as a dicarboxylic acid linker, multiblock copolymers having an average molecular weight of 98,000 were synthesized in accordance with the same methods as used in Preparation Example 1 and named MBP-29. Through the peak generation near 4.2 ppm and terminal peak analysis due to the introduction of dicarboxylic acid using nuclear magnetic resonance, the produced multiblock copolymers were identified as the poloxamers linked by dicarboxylic acid.

### Preparation Example 6. Synthesis of Multiblock Copolymers Composed of Poloxamer 188 Using Sebacoyl Dichloride

With the exception that Poloxamer 188 (molecular weight: 8,000 daltons, PEO:PPO=80:27) was used as a basic unit of multiblock copolymer and sebacoyl dichloride was used as a dicarboxylic acid linker, multiblock copolymers having an average molecular weight of 124,000 were synthesized in accordance with the same methods as used in Preparation Example 1 and named MBP-22. Through the peak generation near 4.2 ppm and terminal peak analysis due to the introduction of dicarboxylic acid using nuclear magnetic resonance, the produced multiblock copolymers were identified as the poloxamers linked by dicarboxylic acid.

### Preparation Example 7. Synthesis of Multiblock Copolymers Composed of Poloxamer 407 Using Dodecan Dichloride

With the exception that Poloxamer 407 (molecular weight: 12,500 daltons, PEO:PPO=101:56) was used as a basic unit of multiblock copolymer and dodecandioyl chloride was used as a dicarboxylic acid linker, multiblock copolymers having an average molecular weight of 110,000 were synthesized in accordance with the same methods as used in Preparation Example 1 and named MBP-42. Through the peak generation near 4.2 ppm and terminal peak analysis due to the introduction of dicarboxylic acid using nuclear magnetic resonance, the produced multiblock copolymers were identified as the poloxamers linked by dicarboxylic acid.

### Preparation Example 8. Synthesis of Multiblock Copolymers Composed of Poloxamer 407 Using Succinyl Dichloride

The same methods as used in Preparation Example 1 were carried out using Poloxamer 407 (molecular weight: 12,500 daltons, PEO:PPO=101:56) as a basic unit of multiblock copolymer and using succinyl dichloride as a dicarboxylic acid linker, thereby synthesizing multiblock copolymers having an average molecular weight of 120,000, which was named MBP-77.

### Example 1: Determination of Properties of Multiblock Copolymers

### (1) Determination of Gelation Temperature According to the Type of Polymers

The multiblock copolymers synthesized in Preparation Examples 1 to 8 were dissolved in a distilled water to prepare solutions with the concentrations as described in Table 1 below. As controls, Poloxamer 407 (Pluronic® F-127) and Poloxamer 188 (Pluronic® F-68) solutions were prepared. The gelation temperatures of the multiblock copolymer solutions prepared in Preparation Examples 1 to 8 and the control solutions according to the concentrations are shown in Table 1.

**Table 1**

| Multiblock Copolymer | | Molecular Weight (Mw) | Concentration (wt%) | Gelation Temperature (°C) |
|---|---|---|---|---|
| Control Group | Poloxamer 407 (Pluronic® F-127) | ≈ 12,500 | 15 % | -- |
| | | | 25 % | 20 |
| | | | 30 % | 10 |
| | Poloxamer 188 ((Pluronic® F-68) | ≈ 8,000 | 15 % | -- |
| | | | 30 % | -- |
| Preparation Example 1 (MBP-36) | | ≈ 230,000 | 3 % | 28-30 |
| | | | 5 % | 26 |
| | | | 7 % | 24 |
| | | | 10 % | 21∼22 |
| | | | 15 % | 17∼18 |
| Preparation Example 2 | | ≈ 160,000 | 10 % | 35 |
| | | | 15 % | 28 |
| | | | 20 % | 25 |
| Preparation Example 3 | | ≈ 150,000 | 10 % | 28 |
| | | | 15 % | 22-26 |
| | | | 20 % | 18-22 |
| Preparation Example 4 (MBP-53) | | ≈ 130,000 | 10 % | 30 |
| | | | 15 % | 25∼27 |
| | | | 20 % | 21∼22 |
| Preparation Example 5 (MBP-29) | | ≈ 98,000 | 10 % | 30 |
| | | | 15 % | 24 |
| | | | 20 % | 23 |
| | | | 30 % | 17 |
| Preparation Example 6 (MBP-22) | | ≈ 124,000 | 10 % | 28 |
| | | | 15 % | 22∼26 |
| | | | 20 % | 20∼23 |
| Preparation Example 7 (MBP-42) | | ≈ 110,000 | 5 % | 36~40 |
| | | | 10 % | 28 |
| | | | 15 % | 20~24 |
| Preparation Example 8 (MBP-77) | | ≈ 120,000 | 5 % | 26 |
| | | | 7 % | 24 |
| | | | 10 % | 21 |

As seen from the results in Table 1 above, in the case of the control groups, properties representing phase transition temperature such as distinct gelation point at concentrations less than 25% were hardly found whereas the multiblock copolymer solutions of the invention showed gelation points representing distinct phase transition at concentrations even less than 20%.

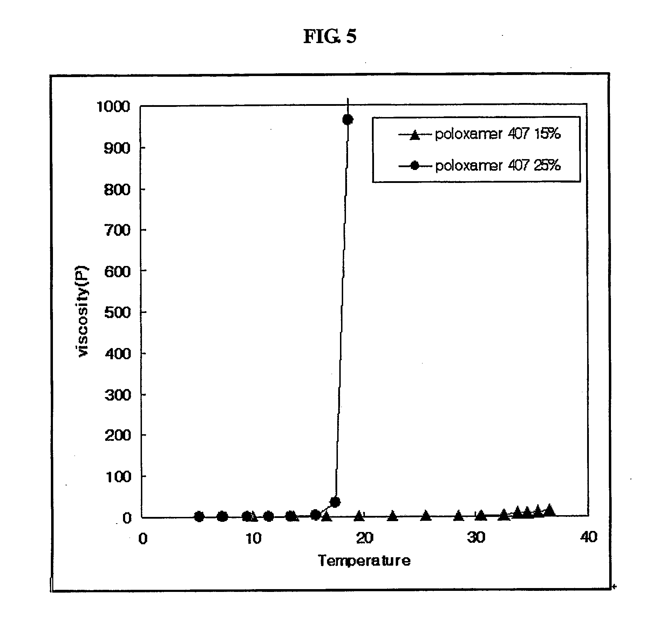

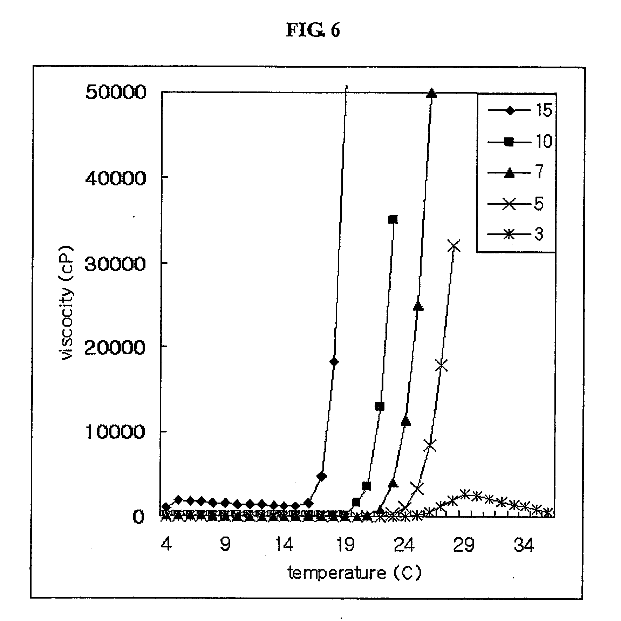

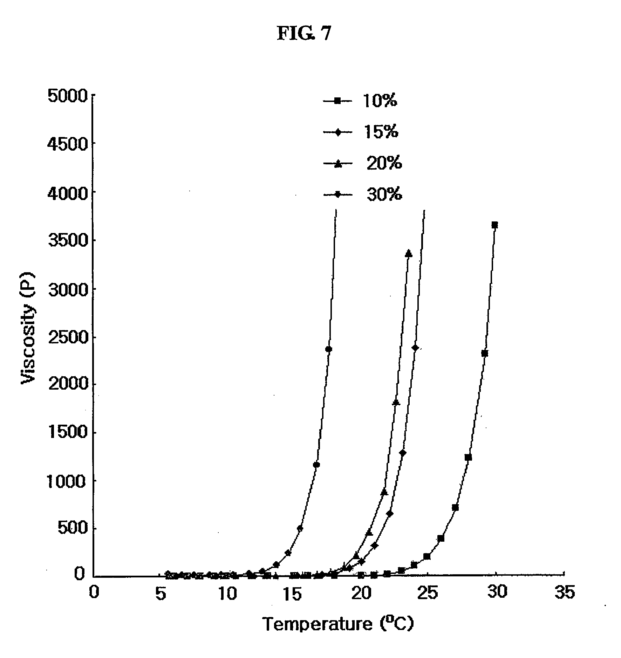

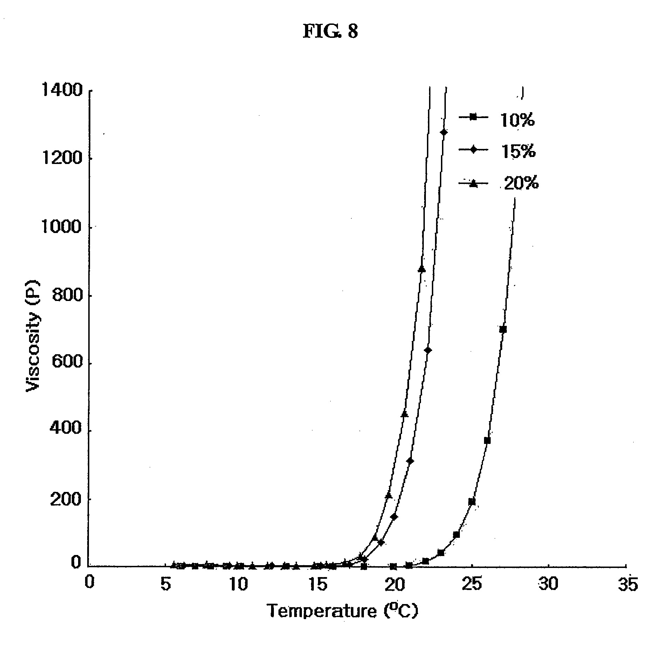

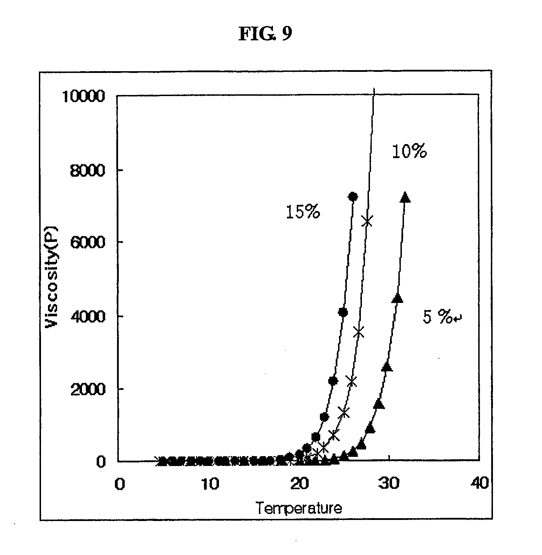

Also, viscosity according to concentrations in Poloxamer 407 solution of the control group solutions and the multiblock copolymer solutions prepared in Preparation Examples 1, and 5 to 7 was determined using Brookfield viscometer and the results were shown in Fig. 5 to Fig. 9, respectively. As can be seen in the above, the viscosity determination results obtained from Brookfield showed correlation with the gelation points obtained using the gradient methods.

### (2) Determination of Gelation Temperature According to the Type of Solvents

In order to examine the properties of polymer aqueous solutions in the case that a mixed solvent of ethanol and distilled water was used as a solvent of aqueous solution, the solvents having the compositions shown in Table 2 were prepared.

**Table 2**

| Multiblock Copolymer | Concentration (wt%) | Molecular Weight (Dalton) | Multiblock Copolymer (g) | Mixing Solvent (g) | Ethanol: Distilled Water (V/V) |
|---|---|---|---|---|---|
| MBP-77 of Preparation Example 8 | | | | | 0 : 100 |
| | 7 | 120,000 | 3.5 | 46.5 | 5 : 95 |
| | | | | | 10 : 90 |
| | | | | | 15 : 85 |
| | | | | | 20 : 80 |
| | | | | | 25 : 75 |

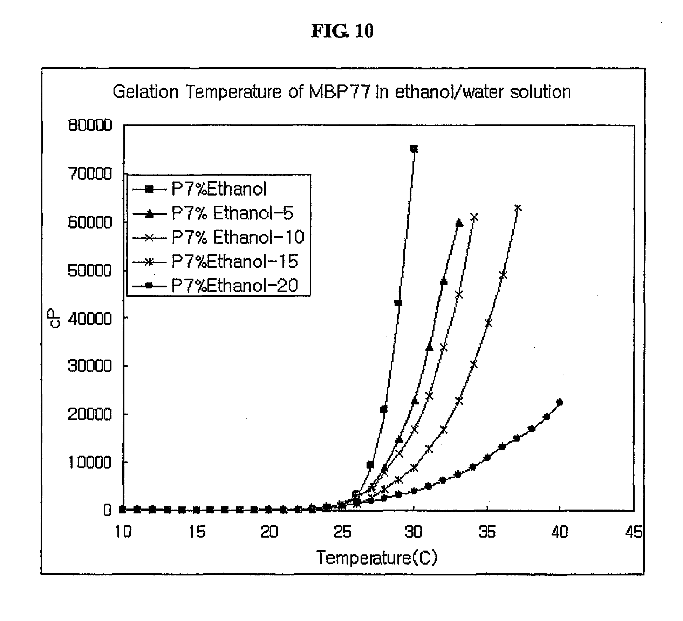

The results of viscosity determination of the ethanol containing aqueous solutions prepared above are shown in Fig. 10.

In the ethanol-free aqueous solution, the viscosity of the solution showed sudden phase transition at a specific temperature as temperature increased whereas in the ethanol containing solvents, it was observed that the viscosity of the solutions had phase transition curves showing gelation points over some broad temperature ranges as the content of ethanol increased.

### Example 2: Determination of Degree of Adhesion of Adhesion Inhibitors Comprising Multiblock Copolymers

In order to examine the adhesion inhibiting effects of the multiblock copolymer solutions prepared above, the degree of adhesion was investigated through animal experiment using SD rats as an animal model. The rats used in this experiment were 6 weeks or older and they were raised separately from another in environments where the temperature of 16 to 22°C and relative humidity of 50 to 70% or so were maintained. After the rats were anesthetized, the abdomen of the anesthetized rats was cut, wound of 1.5 cm x 1.5 cm was artificially formed at epidermal portions of the abdominal walls using scalpel, appendix in contact with this wound was wounded to the extent that its epidermis was slightly peeled off, and the wounded portions were sutured with sutures for surgery. After the multiblock copolymer solutions prepared above were coated onto the sutured region in the amount of 0.5 ∼ 5 ml depending on their concentrations, the degree of tissue adhesion and the weight of the rats were determined on 7 days. Those treated with no solutions were used as a control and compared for adhesion inhibiting effects.

The degree of tissue adhesion was evaluated using the following method [Rodgers et al. (1990)].

### **: Semiquantitative Grading Scale for Tendon Adhesions Grade Evaluation

1: No adhesion observed
2: Separable, filmy adhesion
3: Non-separable, mild adhesion
4: Moderate adhesion (adhesion over 35 to 60% of wounded regions)
5: Severe adhesion (adhesion over regions in excess of 60% of wounded regions)

### (1) Solutions Comprising Multiblock Copolymers (MBP-36) of Preparation Example 1

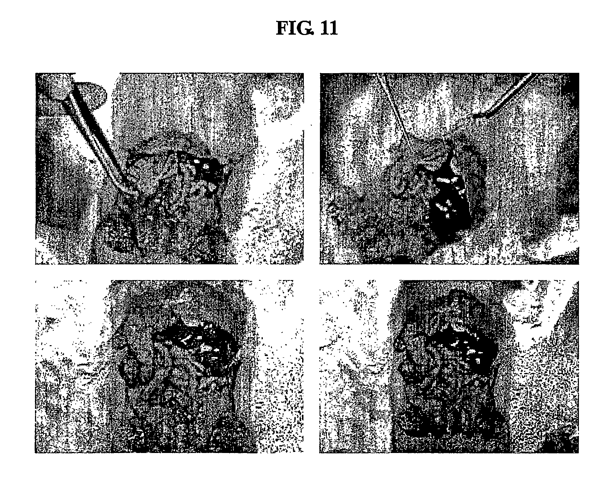

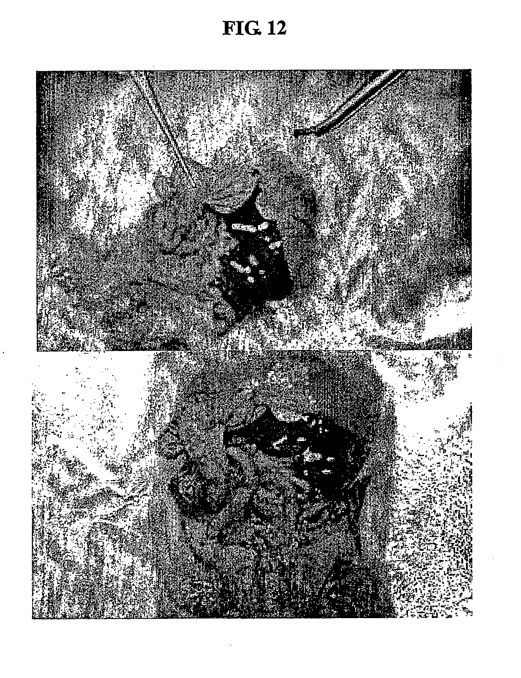

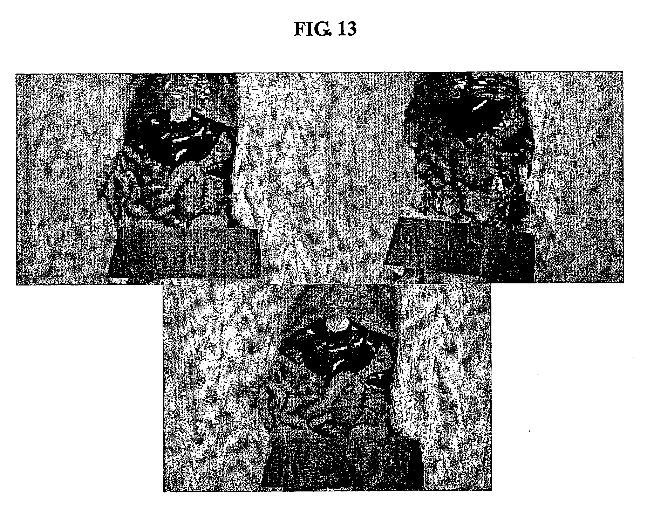

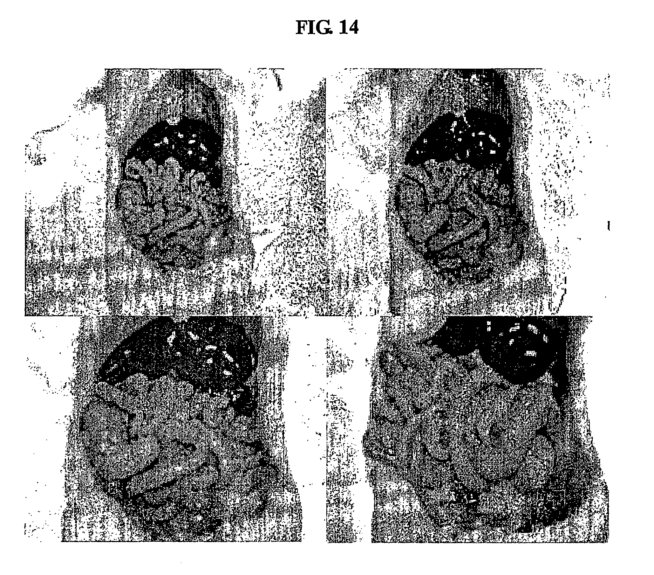

The multiblock copolymer MBP-36 synthesized in Preparation Example 1 was dissolved in physiological saline to prepare solutions with the concentrations as shown in Table 3 below and then the degree of adhesion was determined and the results were shown in Table 3 and Fig. 12 to Fig. 14. The controls treated with no polymers showed severe adhesion at their abdomen and appendix with wounds (Fig. 11), whereas the multiblock copolymers of the invention showed improved adhesion inhibiting effects (Fig. 12 to Fig. 14). Although partial adhesion inhibiting effects were obtained at low concentrations, adhesion was inhibited 100% at concentrations of 5 % or higher.

**Table 3**

| | Concentration (wt%) | Adhesion Inhibition (%) | Population (Number) | | Degree of Adhesion (Population) ** | | | | |
|---|---|---|---|---|---|---|---|---|---|
| | | | Number of Treatment | Number of Adhesion | 1 | 2 | 3 | 4 | 5 |
| MBP-36 | 1 | 0 | 5 | 5 | | | | | 5 |
| | 3 | 20 | 5 | 4 | 1 | 2 | 2 | | |
| | 5 | 100 | 5 | 0 | 5 | - | | | |
| | 7 | 100 | 5 | 0 | 5 | | | | |
| | 10 | 100 | 5 | 0 | 5 | | | | |
| Control Group | | 0 | 5 | 5 | - | - | - | - | 5 |

### (2) Solutions Comprising Multiblock Copolymer MBP-53 of Preparation Example 4

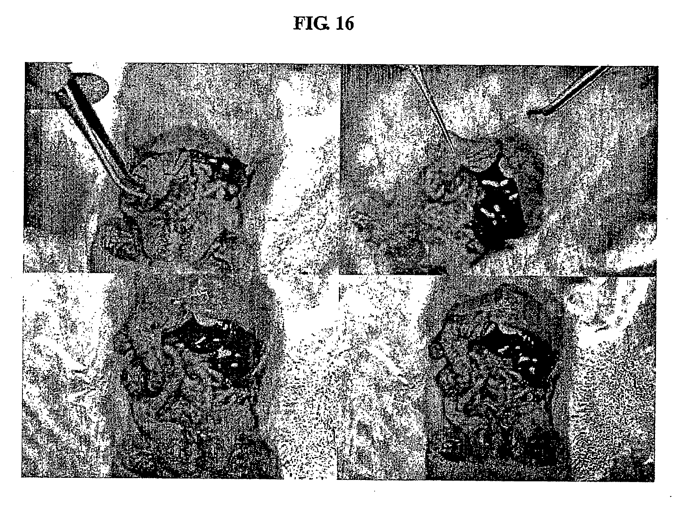

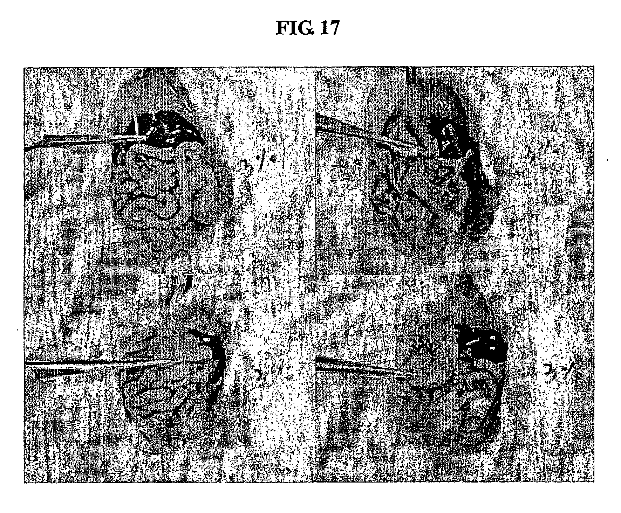

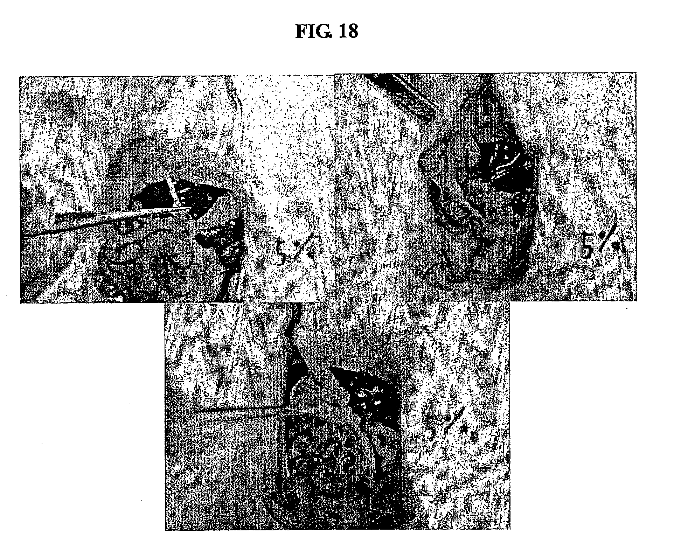

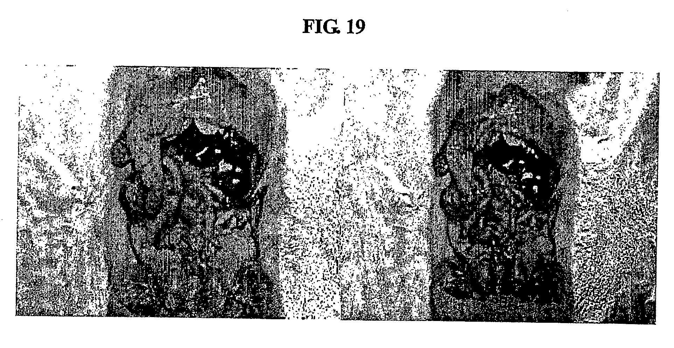

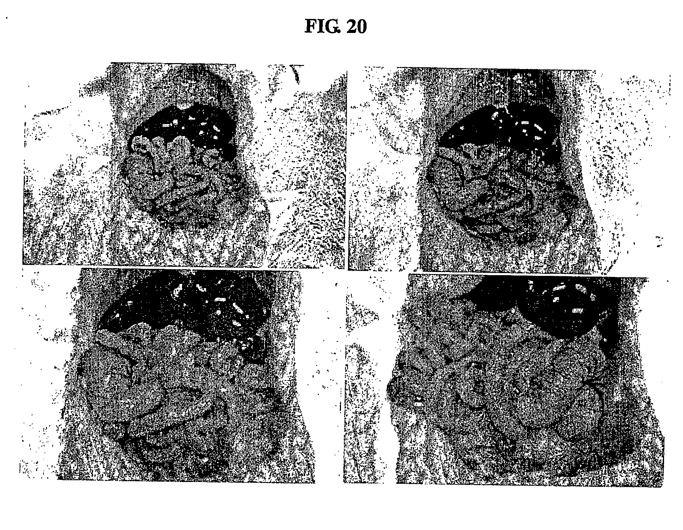

The multiblock copolymer MBP-53 synthesized in Preparation Example 4 was dissolved in physiological saline to prepare solutions with the concentrations as shown in Table 4 below and then the degree of adhesion was determined and the results were shown in Table 4 and Fig. 16 to Fig. 20 (Fig. 16 (1%), Fig. 17 (3%), Fig. 18 (5%), Fig. 19 (7%) and Fig. 20 (10%)). The multiblock copolymer MBP-53 inhibited adhesion 100 % at concentrations of 10 % or higher, it could be seen, from the fact that no remaining MBP-53 was observed, that the applied MBP-53 was all absorbed (Fig. 20), and adhesion inhibiting effects were observed even at low concentrations.

**Table 4**

| | Concentration (wt%) | Adhesion Inhibition (%) | Population (Number) | | Degree of Adhesion (Population) ** | | | | |
|---|---|---|---|---|---|---|---|---|---|
| | | | Number of Treatment | Number of Adhesion | 1 | 2 | 3 | 4 | 5 |
| MBP-53 (Molecular Weight: 13,000 Daltons) | 1 | 0 | 5 | 5 | 0 | | | 5 | |
| | 3 | 0 | 5 | 5 | 0 | 2 | 3 | | |
| | 5 | 40 | 5 | 3 | 2 | 3 | | | |
| | 7 | 60 | 5 | 2 | 3 | 2 | | | |
| | 10 | 100 | 5 | 0 | 5 | - | - | - | - |
| Control Group | | 0 | 5 | 5 | - | - | - | - | 5 |

### (3) Ethanol Containing Aqueous Solutions Comprising Multiblock Copolymer MBP-77 of Preparation Example 8

The multiblock copolymer MBP-77 prepared in Preparation Example 8 was dissolved in a mixing solvent of ethanol and distilled water in accordance with the compositions as shown in Table 5 and then the degree of adhesion was determined and the results were shown in Table 5. The MBP-77 dissolved in the mixed solvent exhibited 100% adhesion inhibiting effects, generally showing possibility of adhesion inhibition. Furthermore, as the content of ethanol increased, the solution was easy to handle during treatment.

**Table 5**

| | Solvent Composition Ethanol: Distilled Water | Adhesion Inhibiting Efficiency (%) | Population (Number) | | Degree of Adhesion | | | | |
|---|---|---|---|---|---|---|---|---|---|
| | | | Number of Treatment | Number of Adhesion | 1 | 2 | 3 | 4 | 5 |
| MBP-77 (Concentrati on 7 wt%) | 1 : 9 | 100 | 5 | | 5 | | | | |
| | 1.5 : 8.5 | 100 | 5 | | 5 | | | | |
| | 2 : 8 | 100 | 5 | | 5 | | | | |
| Control Group | | 0 | 5 | 5 | | | | | 5 |

### Comparative Example 1: Preparation of Poloxamer 407 Solutions

Poloxamer 407 was dissolved in physiological saline shown below to prepare solutions with the concentrations as shown in Table 6 below.

**Table 6**

| Multiblock Copolymer | Molecular Weight | Concentration (wt%) | Multiblock Copolymer (g) | Physiological Saline (g) |
|---|---|---|---|---|
| Poloxamer407 (Pluronic ® F-127) | 12,500 | 5 | 2.5 | 47.5 |
| | | 10 | 5.0 | 45.0 |

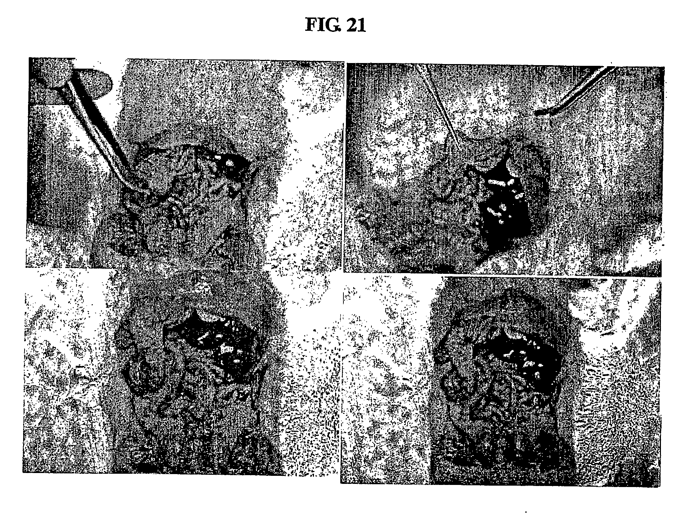

The adhesion inhibiting determination results obtained from animal test using the poloxamer 407 containing solutions were shown in Fig. 21. As seen in Fig. 21, when animals were treated with the Poloxamer 407 solutions, they were not secured within abdominal walls due to their low viscosity unlike gels, flowed like water and less retained within determined positions and importantly, they showed no adhesion inhibiting effects.

### Comparative Example 2: Preparation of Multiblock Copolymer Solutions with Carboxylmethylcellulose Added

The multiblock copolymer MBP-36 prepared in Preparation Example 1 was dissolved in physiological saline, to which carboxylmethylcellulose (CMC) was added in the amount of 1 % by weight, thereby to prepare solutions with the concentrations as shown in Table 7 below.

**Table 7**

| Multiblock Copolymer | Concentration (wt%) | Molecular Weight | Multiblock Copolymer (g) | Physiological Saline (g) | CMC (g) |
|---|---|---|---|---|---|
| MBP-36 | 5 | 230,000 | 2.5 | 47.5 | 0.5 |
| | 10 | | 5.0 | 45.0 | 0.5 |

The degree of adhesion was determined in rats using the above solutions in accordance with the same methods as used in Example 2 and the results are shown in Table 8.

**Table 8**

| Solution Composition | Multiblock Copolymer Concentration (wt%) | Adhesion Inhibition (%) | Population (Number) | | Degree of Adhesion (Population) ** | | | | |
|---|---|---|---|---|---|---|---|---|---|
| | | | Number of Treatment | Number of Adhesion | 1 | 2 | 3 | 4 | 5 |
| MBP-36 + CMC | 5 | 0 | 5 | 0 | 4 | 1 | | | |
| | 10 | 100 | 5 | 0 | 5 | - | | | |

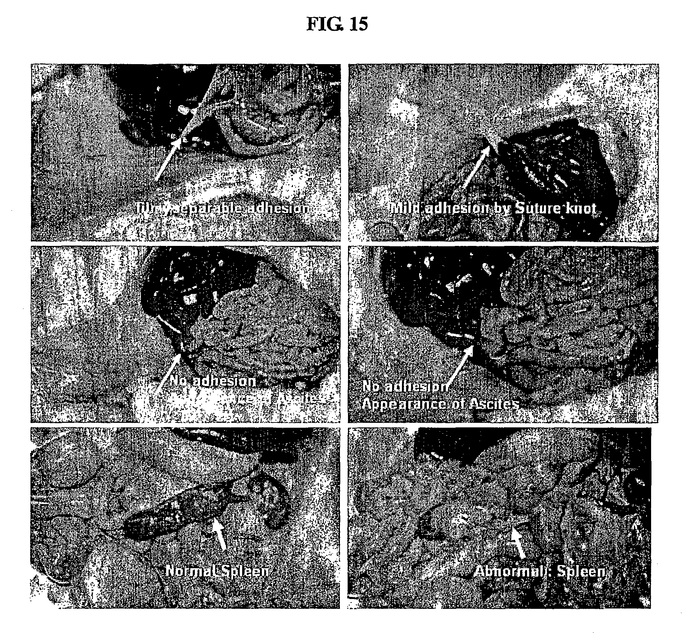

The multiblock copolymer MBP-36 prepared in Preparation Example 1 and carboxymethylcellulose that was reported to have excellent bio-adhesion were dissolved in physiological saline, thereby to prepare solutions where the concentrations of MBP-36 were 5 and 10 wt% and the concentration of carboxylmethylcellulose was 1 wt% and then, adhesion inhibiting effects were determined in rats in accordance with the same methods as used in Example 2 and the results are shown in Fig. 15. When a small amount of carboxylmethylcellulose was added, partial adhesion inhibiting effects were obtained but it has the side effects that spleen exhibited significant overgrowth and some of the animals died. These results propose that carboxylmethylcellulose induced some foreign body reactions.

### Example 3. Change in Weight of the Animals Treated with Adhesion Inhibiting Solutions

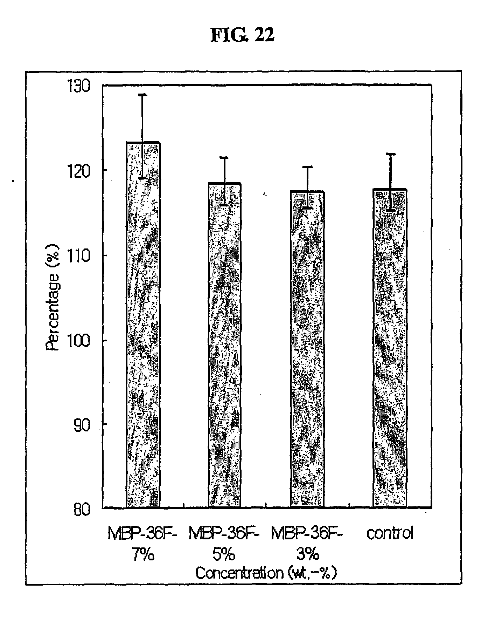

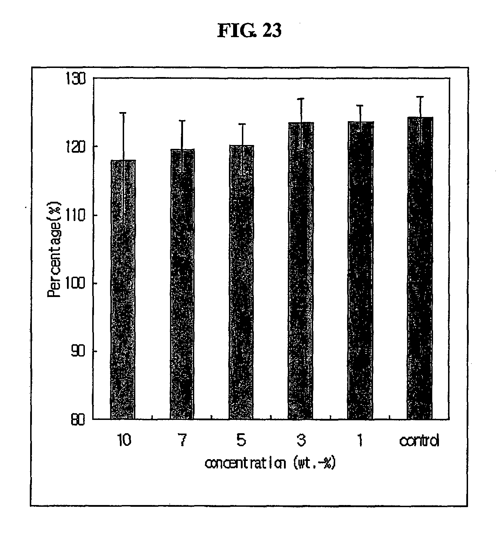

As another test to verify the effectiveness of the compositions for inhibiting adhesion, the weight of the rats treated using the same solutions and methods as used in Example 2 (1) and (2) was observed for one week and the results are shown in Fig. 22 (MBP-36) and Fig. 23 (MBP-53). Those treated with no solutions were used as a control. The weight of the animals treated with the compositions for inhibiting adhesion increased stably, like the control and this indicates that the compositions for inhibiting adhesion are suitable and safe for use in living body.

The polymers of the invention are gelated by body temperature and retained safely within organs for a certain period of time, functioning as an adhesion inhibitor and also, they can be absorbed into body or degraded and after degradation in living body, the degradation products can be also easily absorbed into body and excreted and at the same time, the substances generated from the degradation process function to inhibit adhesion as a surfactant, thereby enabling continuous adhesion inhibiting effects for a long time. In addition, foreign body sensation in living body can be minimized and it is simple and convenient to handle them as they can be regionally applied to wounded regions by spraying or coating during surgery and/or after surgery.

## Claims

1. Use of a water-soluble and temperature-sensitive polymer showing a sol-gel phase transition dependant upon temperature in manufacturing a medicament for inhibiting adhesion of tissues,
wherein the polymer is a temperature-sensitive multiblock copolymer where two or more identical or different block copolymers selected from polyethylene oxide (PEO)-polypropylene oxide (PPO)-polyethylene oxide (PEO) block copolymer and polyethylene oxide (PEO)-polybutylene oxide (PBO)-polyethylene oxide (PEO) block copolymer are linked by a biodegradable dicarboxyl group linker,
wherein said multi-block copolymer has an ionic group selected from the group consisting of a monovalent and divalent salt of -SO₃⁻, -PO₃²⁻, or -C(=O)-R-C(=O)-O- at both terminal ends, wherein R is -(CH₂)ₘ- or an aryl of C_{m'}, m is an integer of 0 to 20, and m' is an integer of 6 to 12,
wherein the polymer is subjected to phase-transition from a sol state to a gel state by the body temperature, stably coated within tissues, thereby functions as a barrier inhibiting adhesion, and as time lapses, degraded into a low molecular weight surfactant and a biodegradable single-molecular substance, whereby the degraded products exhibit secondary adhesion inhibiting effects.

2. Use according to claim 1, wherein the block copolymer has a weight average molecular weight of 1,000 to 20,000 daltons, and the multiblock copolymer has a total weight average molecular weight of 25,000 to 1,000,000 daltons.

3. Use according to claim 1, wherein the block copolymer has a weight average molecular weight of 1,000 to 20,000 daltons, and the multiblock copolymer has a total weight average molecular weight of 50,000 to 500,000 daltons.

4. Use according to claim 1, wherein the multiblock copolymer has a sol-gel phase transition temperature within the range of 10 to 35°C at a concentration of 0.1 to 40 % by weight.

5. Use according to claim 1, wherein the content of the multiblock copolymer is 0.1 to 40 % by weight.

6. Use according to claim 1, wherein the content of the multiblock copolymer is 1 to 20 % by weight.

7. Use according to claim 1, wherein the polymer is used in the form of a composition comprising one selected from the group consisting of a distilled water, water for injection, physiological saline, 0.1 to 50 v/v% alcohol aqueous solution, natural intraperitoneal solution and artificial intraperitoneal solution having the compositions of the natural intraperitoneal solution.

8. Use according to claim 1,
wherein in the block copolymer,
the polyethylene oxide (PEO) block comprises 2 to 2,000 ethylene oxides, the number of each ethylene oxides constituting the two polyethylene oxides included in the respective block copolymers is the same or different from each other,
the polypropylene oxide (PPO) block comprises 2 to 2,000 propylene oxides, and
the polybutylene oxide (PBO) block comprises 2 to 2000 butylene oxides.

9. Use according to claim 1, wherein the dicarboxyl group linker is one or more selected from the group consisting of oxalic acid, succinic acid, glutaric acid, adipic acid, sebacoyl acid, malonic acid, suberic acid, dodecanonic acid, fumaric acid, maleic acid, phthalic acid, and terephthalic acid.

10. Use according to claim 1, wherein the multiblock copolymer has a structure of formula 2:
<formula 2> M-X-O-[PEO-Y-PEO-C(=O)-R-C(=O)-O]ₙ-PEO-Y'-PEO-O-X-M
wherein, PEO is a polyethylene oxide;
Y and Y' are each independently polypropylene oxide (PPO), polybutylene oxide (PBO), or a combination of PPO and PBO;
X is -SO₃⁻, -PO₃²⁻, or -C(=O)-R-C(=O)-O⁻;
n is an integer of 1 to 100;
R is -(CH₂)ₘ-, or an aryl of C_{m'};
m is an integer of 0 to 20, and m' is an integer of 6 to 12; and
M is a monovalent or divalent cation group.

11. Use according to claim 1, wherein the polymer is combined with one or more pharmaceutical drugs selected from the group consisting of anti-thrombogenesis agents, non-steroid anti-inflammatory drugs, hormone chemostatic factors, analgesics and anesthetics.

12. Use according to claim 1, wherein the polymer is applied after laparotomy, laparoscopic surgery, peritoneal surgery, bladder surgery, gynecological surgery, spine surgery, heart surgery, rectal surgery, dental surgery or plastic surgery.

13. Use according to claim 1, wherein the polymer is formulated in the form of a tube, cream, syringe or spray.

14. Use according to claim 1, wherein the multiblock copolymer is degraded in body and continuously releases a polyethyelene oxide (PEO)-polypropylene oxide (PPO)-polyethylene oxide (PEO) block copolymer or polyethyelene oxide (PEO)-polybutylene oxide (PBO)-polyethylene oxide (PEO) block copolymer, thereby exhibiting secondary adhesion inhibiting effects.

## Patentansprüche

1. Verwendung eines wasserlöslichen und temperaturempfindlichen Polymers, das einen temperaturabhängigen Sol-Gel-Phasenübergang zeigt, bei der Herstellung eines Medikaments zur Inhibierung der Adhäsion von Geweben,
wobei es sich bei dem Polymer um ein temperaturempfindliches Multiblockcopolymer handelt, wobei zwei oder mehr gleiche oder verschiedene Blockcopolymere, die aus Polyethylenoxid(PEO)-Polypropylenoxid(PPO)-Polyethylenoxid(PEO)-Blockcopolymer und Polyethylenoxid(PEO)-Polybutylenoxid(PBO)-Polyethylenoxid(PEO)-Blockcopolymer ausgewählt sind, durch einen biologisch abbaubaren Dicarboxylgruppen-Linker verknüpft sind,
wobei das Multiblockcopolymer eine ionische Gruppe aus der Gruppe bestehend aus einem einwertigen und zweiwertigen Salz von -SO₃⁻, -PO₃²⁻ oder -C(=O)-R-C(=O)-O⁻ an beiden terminalen Enden aufweist, wobei R für -(CH₂)ₘ- oder ein Aryl von C_{m'} steht, m für eine ganze Zahl von 0 bis 20 steht und m' für eine ganze Zahl von 6 bis 12 steht,
wobei das Polymer durch die Körpertemperatur einem Phasenübergang von einem Sol-Zustand zu einem Gel-Zustand unterworfen wird, stabil in Geweben beschichtet wird, dadurch als adhäsions-inhibierende Barriere fungiert und im Lauf der Zeit zu einem niedermolekularen Tensid und einer biologisch abbaubaren einzelmolekularen Substanz abgebaut wird, wodurch die abgebauten Produkte sekundäre adhäsionsinhibierende Wirkungen zeigen.

2. Verwendung nach Anspruch 1, wobei das Blockcopolymer ein gewichtsmittleres Molekulargewicht von 1000 bis 20.000 Dalton aufweist und das Multiblockcopolymer ein gesamtes gewichtsmittleres Molekulargewicht von 25.000 bis 1.000.000 Dalton aufweist.

3. Verwendung nach Anspruch 1, wobei das Blockcopolymer ein gewichtsmittleres Molekulargewicht von 1000 bis 20.000 Dalton aufweist und das Multiblockcopolymer ein gesamtes gewichtsmittleres Molekulargewicht von 50.000 bis 500.000 Dalton aufweist.

4. Verwendung nach Anspruch 1, wobei das Multiblockcopolymer bei der Konzentration von 0,1 bis 40 Gew.-% eine Sol-Gel-Phasenübergangstemperatur im Bereich von 10 bis 35°C aufweist.

5. Verwendung nach Anspruch 1, wobei der Gehalt des Multiblockcopolymers 0,1 bis 40 Gew.-% beträgt.

6. Verwendung nach Anspruch 1, wobei der Gehalt des Multiblockcopolymers 1 bis 20 Gew.-% beträgt.

7. Verwendung nach Anspruch 1, wobei das Polymer in Form einer Zusammensetzung, die ein Element aus der Gruppe bestehend aus destilliertem Wasser, Wasser für Injektionszwecke, physiologischer Kochsalzlösung, einer wässrigen Lösung mit 0,1 bis 50 v/v-% Alkohol, natürlicher intraperitonealer Lösung und künstlicher intraperitonealer Lösung mit den Zusammensetzungen der natürlichen intraperitonealen Lösung umfasst, verwendet wird.

8. Verwendung nach Anspruch 1,
wobei in dem Blockcopolymer
der Polyethylenoxid(PEO)-Block 2 bis 2000 Ethylenoxid-Einheiten umfasst, wobei die Zahl der Ethylenoxid-Einheiten, aus denen die beiden Polyethylenoxide in den jeweiligen Blockcopolymeren aufgebaut sind, jeweils gleich oder voneinander verschieden ist,
der Polypropylenoxid(PPO)-Block 2 bis 2000 Propylenoxid-Einheiten umfasst und
der Polybutylenoxid(PBO)-Block 2 bis 2000 Butylenoxid-Einheiten umfasst.

9. Verwendung nach Anspruch 1, wobei es sich bei dem Dicarboxylgruppen-Linker um einen oder mehrere aus der Gruppe bestehend aus Oxalsäure, Bernsteinsäure, Glutarsäure, Adipinsäure, Sebacoylsäure, Malonsäure, Suberinsäure, Dodecanonsäure, Fumarsäure, Maleinsäure, Phthalsäure und Terephthalsäure handelt.

10. Verwendung nach Anspruch 1, wobei das Multiblockcopolymer eine Struktur der Formel 2 aufweist:
< Formel 2> M-X-O-[PEO-Y-PEO-C(=O)-R-C(=O)-O]ₙ-PEO-Y'-PEO-X-M
wobei PEO für ein Polyethylenoxid steht;
Y und Y' jeweils unabhängig für Polypropylenoxid (PPO), Polybutylenoxid (PBO) oder eine Kombination von PPO und PBO stehen;
X für -SO₃⁻, -PO₃²⁻ oder -C(=O)-R-C(=O)-O⁻ steht;
n für eine ganze Zahl von 1 bis 100 steht;
R für -(CH₂)ₘ- oder ein Aryl von C_{m'} steht;
m für eine ganze Zahl von 0 bis 20 steht und m' für eine ganze Zahl von 6 bis 12 steht; und
M für eine einwertige oder zweiwertige Kationgruppe steht.

11. Verwendung nach Anspruch 1, wobei das Polymer mit einem oder mehreren pharmazeutischen Arzneistoffen aus der Gruppe bestehend aus Antithrombogenesemitteln, nichtsteroidalen Antiphlogistika, hormonchemostatischen Faktoren, Analgetika und Anästhetika kombiniert wird.

12. Verwendung nach Anspruch 1, wobei das Polymer nach Laparotomie, laparoskopischer Chirurgie, Peritonealchirurgie, Blasenchirurgie, gynäkologischer Chirurgie, Wirbelsäulenchirurgie, Herzchirurgie, Rektalchirurgie, Dentalchirurgie oder plastischer Chirurgie aufgebracht wird.

13. Verwendung nach Anspruch 1, bei dem das Polymer in Form eines Schlauchs, einer Creme, einer Spritze oder eines Sprays formuliert wird.

14. Verwendung nach Anspruch 1, wobei das Multiblockcopolymer im Körper abgebaut wird und kontinuierlich ein Polyethylenoxid(PEO)-Polypropylenoxid(PPO)-Polyethylenoxid(PEO)-Blockcopolymer oder Polyethylenoxid(PEO)-Polybutylenoxid(PBO)-Polyethylenoxid(PEO)-Blockcopolymer freisetzt und dadurch sekundäre adhäsionsinhibierende Wirkungen zeigt.

## Revendications

1. Utilisation d'un polymère sensible à la température et soluble dans l'eau présentant une transition de phase solide-gel dépendante de la température pour la fabrication d'un médicament pour inhiber l'adhésion des tissus,
où le polymère est un copolymère multibloc sensible à la température où deux ou plusieurs copolymères blocs identiques ou différents choisis parmi un copolymère bloc d'oxyde de polyéthylène (PEO) - oxyde de polypropylène (PPO) - oxyde de polyéthylène (PEO) et un copolymère bloc d'oxyde de polyéthylène (PEO) - oxyde de polybutylène (PBO) - oxyde de polyéthylène (PEO) sont liés par un lieur de groupe dicarboxyle biodégradable,
où ledit copolymère multibloc a un groupe ionique choisi dans le groupe consistant en un sel monovalent et divalent de -SO₃⁻, -PO₃²⁻, ou -C(=O)-R-C(=O)-O⁻ aux deux extrémités terminales, où R est -(CH₂)ₘ- ou un aryle de C_{m'}, m est un nombre entier de 0 à 20, et m' est un nombre entier de 6 à 12,
où le polymère est soumis à une transition de phase d'un état solide à un état gel par la température du corps, recouvert de façon stable dans les tissus, de ce fait fonctionne comme une barrière inhibant l'adhésion, et comme le temps passe, est dégradé en un tensioactif de faible poids moléculaire et une substance mono-moléculaire biodégradable, par lequel les produits dégradés présentent des effets secondaires d'inhibition de l'adhésion.

2. Utilisation selon la revendication 1, où le copolymère bloc a un poids moléculaire moyen en poids de 1000 à 20000 daltons, et le copolymère multibloc a un poids moléculaire moyen en poids de 25000 à 1000000 daltons.

3. Utilisation selon la revendication 1, où le copolymère bloc a un poids moléculaire moyen en poids de 1000 à 20000 daltons, et le copolymère multibloc a un poids moléculaire moyen en poids de 50000 à 500000 daltons.

4. Utilisation selon la revendication 1, où le copolymère multibloc a une température de transition de phase solide-gel dans la gamme de 10 à 35°C à une concentration de 0,1 à 40% en poids.

5. Utilisation selon la revendication 1, où la teneur en copolymère multibloc est de 0,1 à 40% en poids.

6. Utilisation selon la revendication 1, où la teneur en copolymère multibloc est de 1 à 20% en poids.

7. Utilisation selon la revendication 1, où le polymère est utilisé sous la forme d'une composition comprenant un élément choisi dans le groupe consistant en l'eau distillée, l'eau pour injection, une solution saline physiologique, une solution hydro-alcoolique de 0,1-50% v/v, une solution intrapéritonéale naturelle et une solution intrapéritonéales artificielle ayant les compositions de la solution intrapéritonéale naturelle.

8. Utilisation selon la revendication 1,
où dans le copolymère bloc,
le bloc d'oxyde de polyéthylène (PEO) comprend de 2 à 2000 oxydes d'éthylène, le nombre de chaque oxyde d'éthylène constituant les deux oxydes de polyéthylène inclus dans les copolymères blocs respectifs est le même ou différent l'un de l'autre.
le bloc d'oxyde de polypropylène (PPO) comprend de 2 à 2000 oxydes de propylène, et
le bloc d'oxyde de polybutylène (PBO) comprend de 2 à 2000 oxydes de butylène.

9. Utilisation selon la revendication 1, où le lieur de groupe dicarboxyle est un ou plusieurs choisi(s) dans le groupe consistant en l'acide oxalique, l'acide succinique, l'acide glutarique, l'acide adipique, l'acide sebacoyle, l'acide malonique, l'acide subérique, l'acide dodécanonique, l'acide fumarique, l'acide maléique, l'acide phtalique, et l'acide téréphtalique.

10. Utilisation selon la revendication 1, où le copolymère multibloc a une structure de formule 2 :
<formule 2> M-X-O-[PEO-Y-PEO-C(=O)-R-C(=O)-O]ₙ-PEO-Y'-PEO-O-X-M
où, PEO est un oxyde de polyéthylène ;
Y et Y' sont chacun indépendamment de l'oxyde de polypropylène (PPO), de l'oxyde de polybutylène (PBO), ou une combinaison de PPO et PBO ;
X est -SO₃⁻, -PO₃²⁻, ou -C(=O)-R-C(=O)-O⁻;
n est un nombre entier de 1 à 100 ;
R est -(CH₂)ₘ- ou un aryle de C_{m'} ;
m est un nombre entier de 0 à 20, et m' est un nombre entier de 6 à 12 ; et
M est un groupe cation monovalent ou divalent.

11. Utilisation selon la revendication 1, où le polymère est combiné avec un ou plusieurs médicaments pharmaceutiques choisi(s) dans le groupe consistant en des agents anti-thrombogénèse, des médicaments anti-inflammatoires non-stéroidiens, des facteurs d'hormones chemostatiques, des analgésiques et anesthésiques.

12. Utilisation selon la revendication 1, où le polymère est appliqué après laparotomie, chirurgie laparoscopique, chirurgie péritonéale, chirurgie de la vessie, chirurgie gynécologique, chirurgie de la colonne vertébrale, chirurgie cardiaque, chirurgie rectale, chirurgie dentaire ou chirurgie plastique.

13. Utilisation selon la revendication 1, où le polymère est formulé sous la forme d'un tube, d'une crème, d'une seringue ou d'un spray.

14. Utilisation selon la revendication 1, où le copolymère multibloc est dégradé dans le corps et libère en continu un copolymère bloc d'oxyde de polyéthylène (PEO) - oxyde de polypropylène (PPO) - oxyde de polyéthylène (PEO) ou un copolymère bloc d'oxyde de polyéthylène (PEO) - oxyde de polybutylène (PBO) - oxyde de polyéthylène (PEO), de ce fait présentant des effets secondaires d'inhibition de l'adhésion.
